# EUROPEAN PATENT APPLICATION

(11) **EP 1 709 946 A1**
(43) Date of publication of application: **11.10.2006**
(21) Application number: 05007308.9
(22) Date of filing: 04.04.2005
(51) Int. Cl.: A61F 9/02

(54) **Protective cushion attachable to eyeglasses**

(71) Applicant: Chen, How-Lung, Tainan Hsien, Taiwan, R.O.C. (TW)
(72) Inventor: Chen, How-Lung, Tainan Hsien, Taiwan, R.O.C. (TW)
(74) Representative: Volpert, Marcus

(57) **Abstract**

A protective cushion attached to eyeglasses comprises at least one first fastener piece 21 attached on the inner surface around the eye portions of an eyeglass frame 20 and at least one cushion piece 3 corresponding to the first fastener piece 21. The cushion piece 3 is provided with a second fastener piece 30, which cushion piece 3 is a sponge body 31 enclosed by gas permeable cloth 32. The cushion piece 3 will be attached onto the inner surface around the eye portions of the eyeglass frame 20 for reducing the harm to eyes and the surrounding portions due to an external impact on the eyeglass frame 20 and for keeping the skin in contact of the protective cushion dry and free from eczema. Further, the cushion piece 3 is easy to replace.

## Description

### BACKGROUND OF THE INVENTION

### 1. Field of the Invention

The present invention relates to protective cushions attached to eyeglasses, more particularly to a protective cushion comprising at least one first fastener piece attached on the inner surface around the eye portions of an eyeglass frame and at least one cushion piece corresponding to the first fastener piece. The cushion piece is provided with a second fastener piece, which cushion piece is a sponge body enclosed by gas permeable cloth. The cushion piece will be attached onto the inner surface around the eye portions of the eyeglass frame for reducing the harm to eyes and the surrounding portions due to an external impact on the eyeglass frame and for keeping the skin in contact of the protective cushion dry and free from eczema. Further, the cushion piece is easy to replace.

### 2. Description of the Prior Art

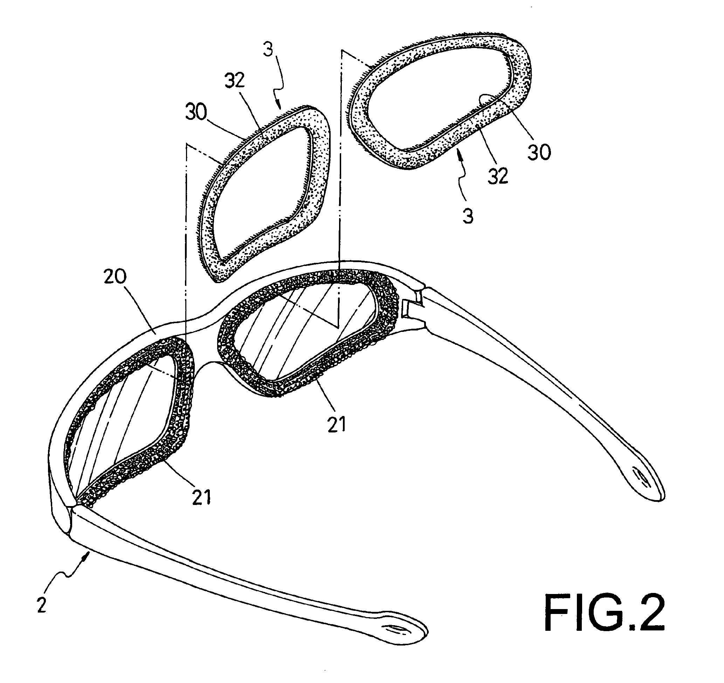

As a hard eyeglass frame of a pair of eyeglasses is punched by an external force, the eyeglass frame may cause harm to the eyes and the surrounding portions of the user. Therefore, an eyeglass frame with protective cushion was invented, as shown in Fig.2, which has a cushion piece 11 made of sponge attached to the inner surface around the eye portions of an eyeglass frame 10 of a pair of eyeglasses 1, whereby harm to the eyes due to an external impact will be prevented. However, the protective cushion attached to eyeglasses of the prior art has at least the following disadvantages: (1) that the sponge body of the protective cushion has low air permeability and therefore may cause eczema to the skin in contact with the cushion; and (2) that the cushion piece 11 of the protective cushion is permanently attached to eyeglasses and cannot be replaced.

### SUMMARY OF THE INVENTION

The primary objective of the present invention is to provide a protective cushion attached to eyeglasses that is easily replaceable and of effective gas permeability.

Accordingly, the first preferred embodiment of the present invention is a protective cushion attached to eyeglasses comprising a pair of first fastener pieces attached on the inner surface around the eye portions of an eyeglass frame and a pair of cushion pieces respectively corresponding to the first fastener pieces, each of the cushion pieces being provided with a second fastener piece and being a sponge body enclosed by gas permeable cloth.

The second preferred embodiment of the present invention is a protective cushion attached to eyeglasses comprising a single first fastener piece attached on the inner surface around the eye portions of an eyeglass frame and a single cushion piece corresponding to the first fastener piece, which cushion piece is provided with a second fastener piece and is a sponge body enclosed by gas permeable cloth.

### BRIEF DESCRIPTION OF DRAWINGS

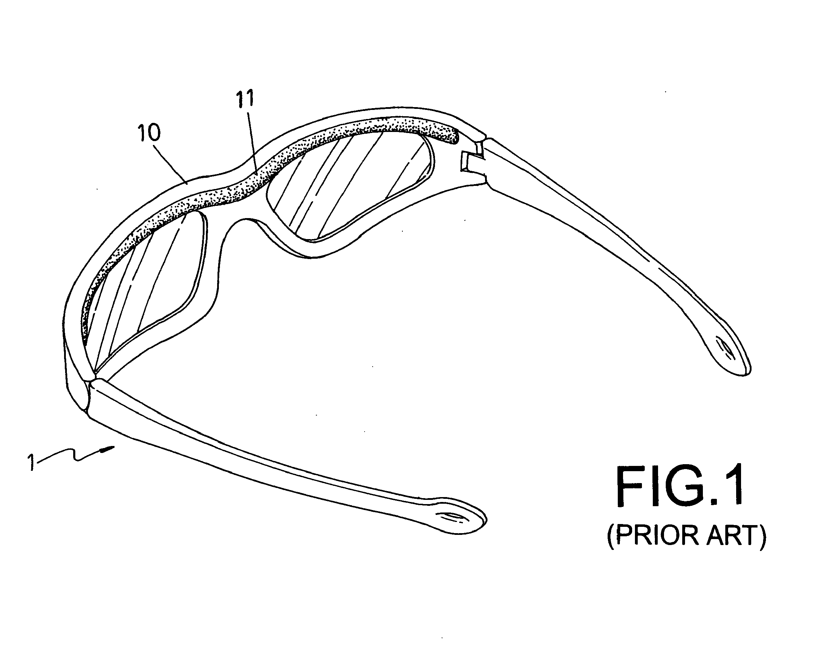

Fig.1 is a perspective view of a protective cushion attached to eyeglasses of the prior art.
Fig.2 is an exploded perspective view of the first preferred embodiment of the present invention as a protective cushion attached to eyeglasses.
Fig.3 is a perspective view of the first preferred embodiment of the present invention.
Fig.4 is a lateral cross-sectional view of the first preferred embodiment of the present invention.
Fig.5 is an exploded perspective view of the second preferred embodiment of the present invention as a protective cushion attached to eyeglasses.
Fig.6 is a perspective view of the second preferred embodiment of the present invention.
Fig.7 is a lateral cross-sectional view of the second preferred embodiment of the present invention.

### DETAILED DESCRIPTION OF THE PREFERRED EMBODIMENTS

The various objects and advantages of the present invention will be more readily understood from the following detailed description when read in conjunction with the appended drawings.

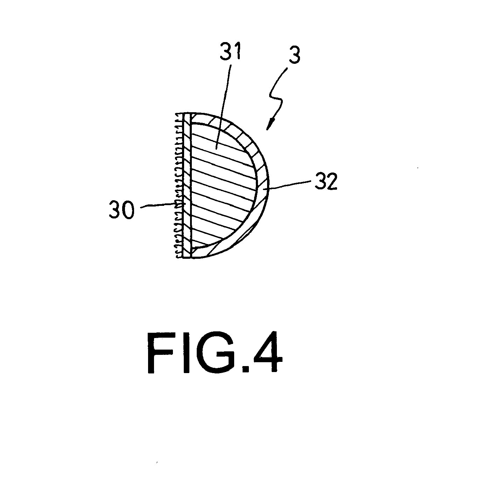

Referring to Fig.2 and 4, a protective cushion attached to eyeglasses according to the present invention comprises a pair of fastener pieces 21 attached on the inner surface of an eyeglass frame 20 of the eyeglasses 2 and a pair of cushion pieces 3 respectively corresponding to the fastener pieces 21. Each of the cushion pieces 3 is provided with a fastener piece 30, which cushion pieces are each a sponge body 31 enclosed by gas permeable cloth 32.

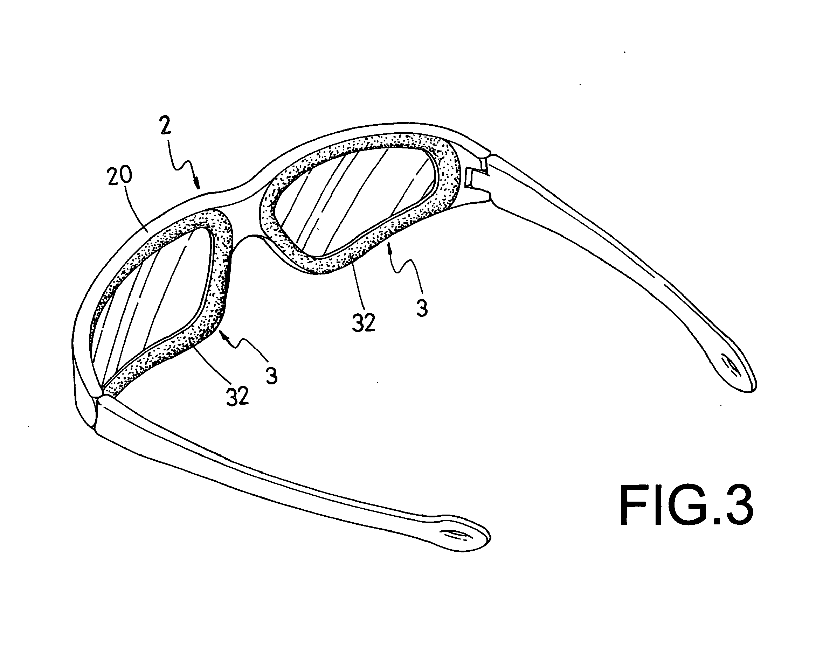

Please refer to Fig.2, 3 and 4 for the use of the protective cushion attached to eyeglasses. Each of the fastener pieces 30 of the cushion pieces 3 is coupled with a corresponding fastener piece 21 on the eyeglass frame 20, whereby the cushion pieces 3 will be attached onto the inner surface around the eye portions of the eyeglass frame 20. Therefore, the installation of the protective cushion is completed. By means of the cushion pieces 3, the harm to eyes and the surrounding portions due to an external impact on the eyeglass frame 20 can be significantly reduced. Further, the gas permeable cloth 32 of the cushion pieces 3 will promote perspiration and air ventilation, whereby the skin in contact of the protective cushion may keep dry and be free from eczema. To replace either of the cushion pieces 3, a cushion piece 3 can be easily removed from the eyeglass frame 20, and a new cushion piece 3 can be installed in the same way described above.

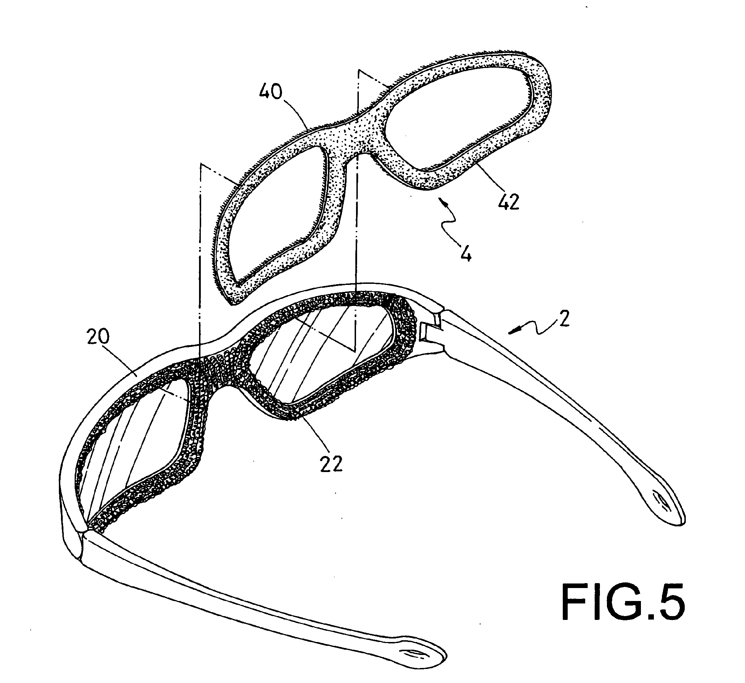

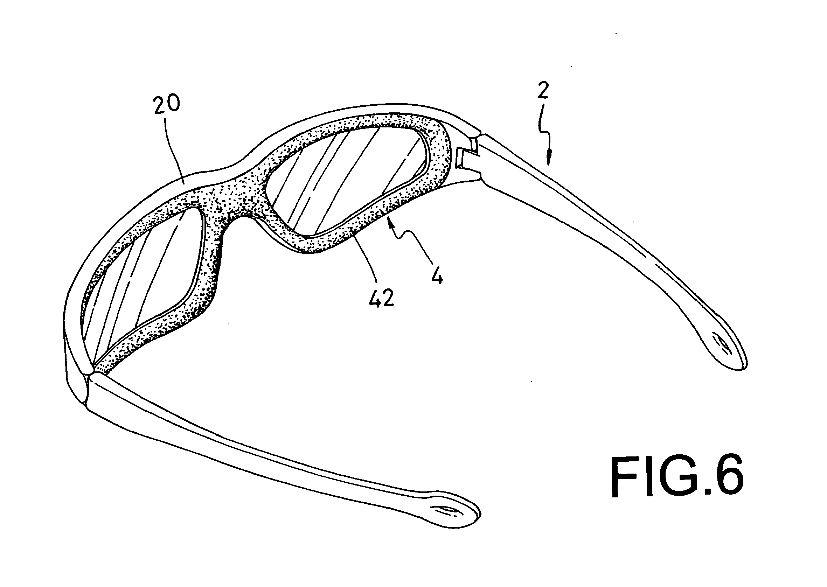

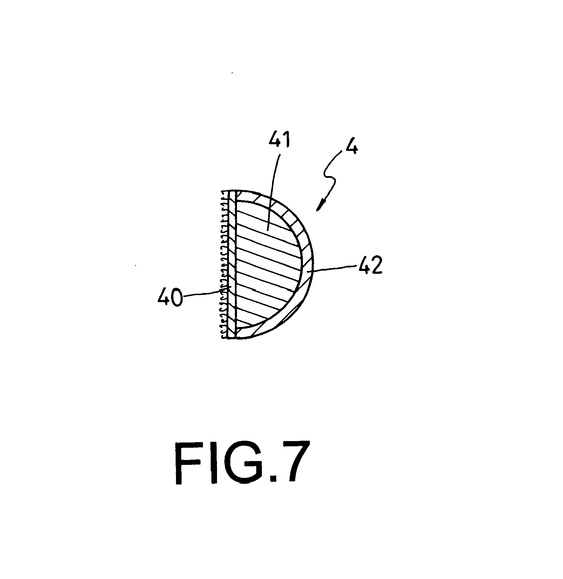

Please refer to Fig.5, 6 and 7 for another preferred embodiment of the present invention. The preferred embodiment comprises a single fastener piece 22 attached on the inner surface of an eyeglass frame 20 and a single cushion piece 4 corresponding to the fastener piece 22. The cushion piece 4 is provided with a fastener piece 40, which cushion piece is a sponge body 41 enclosed by gas permeable cloth 42. To use this preferred embodiment, the fastener piece 40 of the cushion piece 4 is coupled with the fastener piece 22 on the eyeglass frame 20, whereby the cushion piece 4 will be attached onto the inner surface around the eye portions of the eyeglass frame 20. By means of the cushion piece 4, the harm to eyes and the surrounding portions due to an external impact on the eyeglass frame 20 can be significantly reduced. Further, the gas permeable cloth 42 of the cushion piece 4 will promote perspiration and air ventilation, whereby the skin in contact of the protective cushion may keep dry and be free from eczema. To replace the cushion piece 4, it is easily removed from the eyeglass frame 20, and a new cushion piece can be installed in the same way described above.

The present invention is thus described, and it will be obvious that the same may be varied in many ways. Such variations are not to be regarded as a departure from the spirit and scope of the present invention, and all such modifications as would be obvious to one skilled in the art are intended to be included within the scope of the following claims.

## Claims

1. A protective cushion attached to eyeglasses, comprising:
a pair of first fastener pieces attached on an inner surface around eye portions of an eyeglass frame; and
a pair of cushion pieces respectively corresponding to said first fastener pieces, each of said cushion pieces being provided with a second fastener piece, each of said cushion pieces being a sponge body enclosed by gas permeable cloth.

2. A protective cushion attached to eyeglasses, comprising:
a first fastener piece attached on an inner surface around eye portions of an eyeglass frame; and
a cushion piece corresponding to said first fastener piece provided with a second fastener piece, said cushion piece being a sponge body enclosed by gas permeable cloth.
